# EUROPEAN PATENT SPECIFICATION

(11) **EP 0 804 238 B1**
(45) Date of publication and mention of the grant of the patent: **29.09.2004**
(21) Application number: 96900220.3
(22) Date of filing: 12.01.1996
(51) Int. Cl.: A61K 41/00

(54) **METHOD TO PREVENT TRANSPLANT REJECTION**
VERFAHREN ZUR VERHINDERUNG VON TRANSPLANTATABSTOSSUNG
PROCEDE PREVENANT LE REJET DE GREFFES

(30) Priority: 13.01.1995 US 371707
(43) Date of publication of application: 05.11.1997
(73) Proprietor: QLT Inc., Vancouver, British Columbia, V5Z 4H5 (CA); UNIVERSITY OF BRITISH COLUMBIA, Vancouver, British Columbia, V6T 1Z3 (CA)
(72) Inventor: OBOCHI, Modestus, Onuora, Kay, Vancouver, British Columbia V5Z 4K7 (CA); LEVY, Julia, Vancouver, British Columbia V6J 4Z3 (CA)
(74) Representative: W.P. Thompson & Co.
(86) International application number: PCT/CA1996/000019
(87) International publication number: WO 1996/021466

(56) References cited:
- WO-A-95/03814
- US-A- 5 028 594
- US-A- 5 147 289
- SCAND. J. IMMUNOL. (1985), 21(3), 267-73 CODEN: SJIMAX;ISSN: 0300-9475, 1985 GRUNER, S. ET AL 'The influence of hematoporphyrin derivative and visible light on murine skin graft survival, epidermal Langerhans cells and stimulation of the allogeneic mixed leukocyte reaction'
- BIOMED. BIOCHIM. ACTA (1986), 45(5), 649-54 CODEN: BBIADT, 1986 GRUNER, S. ET AL 'The influence of photochemical treatment on immunogenicity and epidermal Langerhans cells of murine skin grafts'
- DATABASE CHEMABS CHEMICAL ABSTRACTS SERVICE, COLUMBUS, OHIO, US AN=123:137610, SIMKIN, GUILLERMO ET AL 'Effect of photodynamic therapy using benzoporphyrin derivative on the cutaneous immune response' & PROC. SPIE-INT. SOC. OPT. ENG. (1995), 2392, 23-33 CODEN: PSISDG;ISSN: 0277-786X, 1995
- DATABASE EMBASE ELSEVIER SCIENCE PUBLISHERS, AMSTERDAM, NL A=95324577, & DERMATOLOGIC CLINICS, vol. 13, no. 4, 1995 pages 739-749, T.W. GRIFFITHS 'IMMUNOPATHOGENESIS AND IMMUNOTHERAPY OF PSORIASIS.'

## Description

### Technical Field

The invention relates to procedures for supplying allografts in therapy and to preventing the rejection of allografts by a recipient. In particular, it concerns the treatment of donor tissue with photodynamic therapy techniques to deplete the tissue of antigen-presenting cells and to diminish the immunogenicity of cells contained therein.

### Background Art

The success of a transplant of an allograft in a host depends on such factors as the antigens on the transplanted tissue that are recognized by the recipient as foreign and can evoke the rejection response, the cells in the recipient's immune system that mediate rejection, and the reactions that modify either the presentation of the foreign antigen or the cellular response. It is known that a significant component of allograft rejection is due to the presence in donor tissue of non-parenchymal cells (passenger leukocytes).

It is also known that the products of the major histocompatibility complex (MHC) play an important role in mediating an attack by the graft tissue against the recipient. The MHC is generally complex because it includes many different loci, each encoding separate cell-surface antigens, and because the loci have extensive polymorphism. The loci of the MHC fall into one of two classes, Class I or Class II, based on their tissue distribution, the structure of the expressed antigens, and their functions. Class I antigens, present on all nucleated cells, serve as the primary targets for cytotoxic T (CD8⁺) lymphocytes. Class II antigens are not distributed in the tissue as widely and serve as primary targets for helper T (CD8⁺) lymphocytes.

The polymorphic forms of the individual loci of human leukocyte antigen (HLA), the MHC in humans, have been recognized by antibodies and by various in vitro techniques that measure T-lymphocyte recognition. These responses, mediated by the recipient's recognition of polymorphism in the donor, correlate with the strong rejection reactions that take place in vivo. Investigation into the cellular basis of graft rejection, using both in vitro and in vivo studies, has revealed that both CD4⁺ and CD8⁺ lymphocytes participate in the rejectión response.

Attempts to prolong the survival of allografts and xenografts after transplantation, both in experimental models and in medical practice, have centered mainly on the suppression of the immune apparatus of the recipient. This treatment has as its aim preventive immunosuppression and/or treatment of graft rejection.

Examples of agents used for immunosuppression include cytotoxic drugs, antimetabolites, corticosteroids, and antilymphocytic serum. Nonspecific immunosuppressive agents found particularly effective in preventive immunosuppression (azathioprine, bromocryptine, methyl prednisolone, prednisone, and cyclosporin A) have significantly improved the clinical success of transplantation. The nephrotoxicity of cyclosporin A after renal transplantation has been reduced by co-administration of steroids such as prednisolone, or prednisolone in conjunction with azathioprine. In addition, kidneys have been grafted successfully using anti-lymphocyte globulin followed by cyclosporin A. Another protocol is total lymphoid irradiation of the recipient prior to transplantation, followed by minimal immunosuppression after transplantation. Treatment of rejection has involved the use of steroids, 2-amino-6-aryl-5-substituted pyrimidines, heterologous anti-lymphocyte globulin, and monoclonal antibodies to various leukocyte populations.

The principal complication of immunosuppressive drugs is infections. Additionally, systemic immunosuppression is accompanied by undesirable toxic effects, e.g., nephrotoxicity when cyclosporin A is used after renal transplantation, and reduction in the level of the hemopoietic stem cells. Immunosuppressive drugs may also lead to obesity, poor wound healing, steroid hypoglycemia, steroid psychosis, leukopenia, gastrointestinal bleeding, lymphoma and hypertension.

In view of these complications, transplantation immunologists have sought methods for suppressing immune responsiveness in an antigen-specific manner so that only the response to the donor alloantigen would be lost. Such specific immunosuppression generally has been achieved by modifying either the antigenicity of the tissue to be grafted or the specific cells capable of mediating rejection. In certain instances, whether immunity or tolerance will be induced depends on the manner in which the antigen is presented to the immune system. Other anti-rejection strategies have focused on the elimination or attenuation of MHC-bearing passenger leukocytes, i.e., antigen-presenting cells ("APC's"), in donor tissues prior to transplant.

Techniques that have been reported for this purpose include extended time culturing of the donor tissue (Lafferty *et al*., "Thyroid Allograft Immunogenicity is Reduced after a Period in Organ Culture", Science, 188:259 (1975)). Pre-treating the allograft tissues by growth in tissue culture before transplantation has been found in two murine model systems to lead to permanent acceptance across MHC barriers (Lafferty et al., Transplantation, 22:138-49 (1976); Bowen et al., Lancet, 2:585-86 (1979)). It has been hypothesized that such treatment results in the depletion of passenger lymphoid cells and thus the absence of a stimulator cell population necessary to tissue immunogenicity. For example, some donor-recipient HLA matching has been used, e.g., in vascular and kidney grafts and sometimes prior to blood transfusions.

Donor tissue has been treated with growth factor, such as TGF-beta (Czarniecki et al., U.S. Patent No. 5,135,915 issued 4 August 1992), sometimes in combination with extended culture times (Orton, U.S. Patent No. 5,192,312 issued 9 March 1993).

Donor tissue may be treated with UV light (Reemtsma et al., U.S. Patent No. 4,946,438 issued 7 August 1990; and Lau *et al*., "Prolongation of Rat Islet Allograft Survival by Direct Ultraviolet Irradiation of the Graft, Science, 223:607 (1984)), sometimes in conjunction with microencapsulation (Weber et al., U.S. Patent No. 5,227,298, issued 13 July 1993). Other workers have used barrier membranes alone, such as the bilayer comprising a first non-cytotoxic layer and a second outer layer of a biocompatible and semipermeable polymeric material taught by Cochrum, U.S. Patent No. 4,696,286 issued 29 September 1987.

Donor tissues have been treated with a wide variety of substances, such as the topical application of cyclosporin to skin grafts, as disclosed by Hewitt et al., U.S. Patent No. 4,996,193 issued 26 February 1991, and the perfusion of a donor kidney with lymphocytic chalone, as described by Jones et al., U.S. Patent No. 4,294,824 issued 13 October 1981. The survival time of skin grafts has been prolonged by treatment in vitro with cortisone, thalidomide, or urethane before implantation into a laboratory animal. The amount of drug locally applied to the skin is usually smaller than the amount required to achieve a similar effect by injecting the drug systemically into the recipient. Donor skin has been treated in vitro with streptokinase/streptodornase, RNA and DNA preparations of the recipient, or solutions of glutaraldehyde, prior to transplantation to reduce the antigenicity of the skin to be grafted.

More sophisticated approaches have involved the treatment of donor tissue with a monoclonal antibody directed against the MHC product along with complement (Faustman *et al*., "Prolongation of Murine Islet Allograft Survival by Pretreatment of Islets with Antibody Directly to Ia Determinants", Proc. Natl. Acad. Sci. USA, 78:5156 (1981)) or the treatment of donor tissue with an immunoconjugate of antibody directed against the MHC (Shizuru, J.A., *et al*., "Inhibition of Rat Mixed Lymphocyte Pancreatic Islet Cultures with Anti-Ia Immunotoxin", Transplantation, 42:660 (1986)). Variable results were obtained by these methods. Thus, there is a need in the art for a method to prolong graft survival in transplant operations that would minimize the toxicity and other adverse effects arising from the use of large doses of immunosuppressants.

The technique employed according the present invention for selectively destroying these APC cells involves contacting donor tissue with a photosensitizer, followed by exposure to light, and then transplantation. Similar photodynamic methods have previously been used primarily for destroying tissues such as tumor tissues, atherosclerotic plaques, surface skin diseases, and unwanted pathogens in blood (Levy et al., U.S. Patent Nos. 5,283,255 issued 1 February 1994; 4,883,790 issued 28 November 1989; 4,920,143 issued 24 April 1990; 5,095,030 issued 10 March 1992; and 5,171,749 issued 15 December 1992). See also, Dougherty *et al*., U.S. Patent Nos. 4,932,934 issued 12 June 1990; 4,889,129 issued 26 December 1989; 5,028,621 issued 2 July 1991; 4,866,168 issued 12 September 1989; 5,145,863 issued 8 September 1992; and 4,649,151 issued 10 March 1987.

For example, U.S. Patent 4,866,168 to Dougherty *et al*. discloses a composition sold under the trademark "Photofrin II", which is obtained by recovering the high aggregate-molecular weight portion of hematoporphyrin derivative. As another specific example, U.S. Patent 4,883,790 to Levy *et al*. discloses the use of a group of related compounds designated "monohydrobenzoporphyrins" for analogous purposes.

In addition, the use of many various photosensitizers of similar structure has been described. See, for example, the derivatives of (1-hydroxyethyl)deuteroporphyrin, hydrophobic hematoporphyrin ethers, and compounds prepared from methyl pheophorbide (Pandey et al., U.S. Patent No. 5,002,962 issued 26 March 1991); pyropheophorbide conjugates (Pandey et al., U.S. Patent No. 5,314,905); bacteriochlorophyll-a derivatives (Dougherty, U.S. Patent Nos. 5,171,741 and 5,173,504); monovinyl and divinyl ether-linked dimers (Ward, U.S. Patent No. 4,961,920); benzoporphyrin derivatives (Allison et al., U.S. Patent No. 5,214,036); dibenzoporphyrin compounds (Dolphin et al., U.S. Patent Nos. 5,308,608 and 5,149,708); the so-called "green" porphyrins, such as monobenzoporphyrin derivatives (Jamieson et al., U.S. Patent Nol 5,087,636); porphyrin compounds containing exocyclic double bonds (Chang et al., U.S. Patent No. 5,064,952); and porfimer sodium compositions (Clauss et al., U.S. patent No. 5,244,914).

In general, these drugs are regarded, in a first approximation, as being interchangeable in their utility with respect to photodynamic therapy.

While photodynamic therapy primarily concerns treatment of tumor cells, additional applications have previously been shown. For example, these photosensitizing drugs can be used in protocols to eliminate atherosclerotic plaques, and in the treatment of blood and of other body fluids to destroy infectious organisms. However, photodynamic therapy has apparently not yet been used to eradicate passenger leukocytes in donor allograft tissue.

It is a particular advantage of the present invention that, unlike therapeutic regimens involving the administration of a photosensitizing drug to an organism, donor tissue may be most appropriately be treated in vitro prior to an actual transplant procedure. In this manner, problems associated with ensuring proper levels of light exposure of, e.g., a conjugate associated with target cells within an organism, are substantially obviated.

Further, the method of the invention results in grafts that are immunologically stable in suitable hosts, biologically functional, and capable of being stored prior to transplantation. Thus, this invention enables the establishment of a bank of photodynamically treated grafts that can be used for short-term storage.

### Disclosure of the Invention

The invention provides a procedure for minimizing the rejection of transplants in animal subjects. Prior to transplantation, donor tissue, which contains antigen-presenting cells (APC's), is contacted with a photosensitizing agent and exposed to light having a wavelength absorbed by the photosensitizing agent for a time sufficient to deplete the APC's. This treatment does not kill keratinocytes but may alter their expression of Class I and/or II antigens, as well as the cytokines they secrete, such that the immunogenicity of the skin itself is diminished.

One embodiment of the present invention is use of a photosensitizing agent to prepare a medicament for diminishing the immunogenicity of donor tissue containing antigen presenting cells (APC's) during photodynamic therapy, under conditions which are not cytotoxic to said APC's, using an amount of photosensitizing agent of 0.1 to 2µg/ml.

The medicament may be used in a method which comprises:
a. contacting the donor tissue with a photosensitizing agent to obtain a modified donor tissue;
b. exposing said modified donor tissue to light having a wavelength absorbed by said photosensitizing agent for a time sufficient to functionally attenuate the APC's in the donor tissue; and
c. transplanting donor tissue containing modulated APC's into the tissue of a recipient.

Another embodiment of the present invention provides a method of treating a donor tissue containing antigen presenting cells (APC's), which method comprises:
(a) contacting the donor tissue with a photosensitizing agent to obtain a modified donor tissue, and
(b) exposing said modified donor tissue to light having a wavelength absorbed by said photosensitizing agent for a time sufficient to functionally attenuate the APC's in the donor tissue, under conditions which are non-cytoxic to said APC's and using an amount of photosensitizer agent of 0.1 to 2µg/ml

According to a further embodiment of the present invention there is provided a composition of matter which comprises a donor tissue from a donor, said donor tissue containing antigen presenting cells (APC's) and being in admixture with a medicament comprising a photosensitizing agent at a concentration of 0.1 to 2µg/ml.

In one embodiment, the photosensitizing agent is in the form of a conjugate comprising a target-specific component to enhance the interaction between the photosensitizing agent and the target APC's. The photosensitizing drug mediates the destruction of the APC's when the allograft is irradiated at a suitable wavelength absorbed by the photosensitizing agent.

### Brief Description of the Drawings

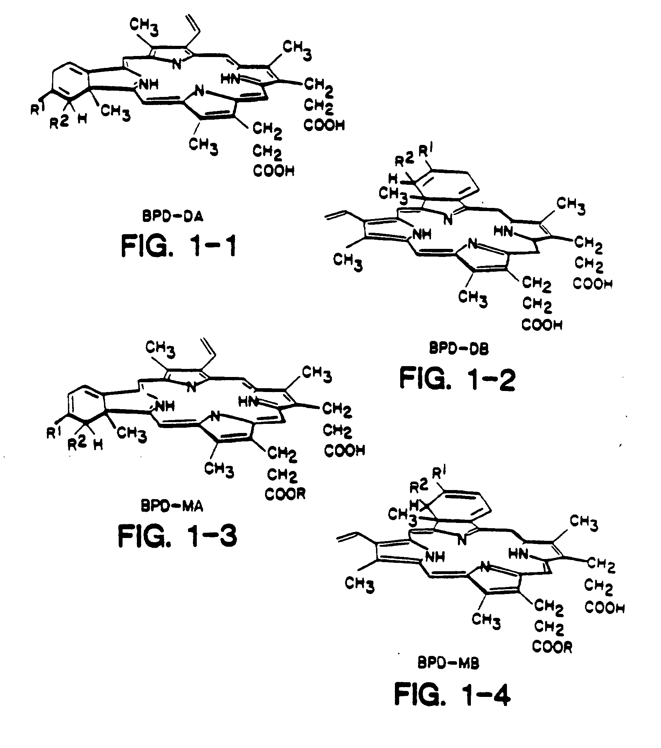

Figure 1 shows the structure of BPD compounds particularly useful as photosensitizing agents in the invention.

### Modes of Carrying Out the Invention

In accordance with the invention described below, prior to transplantation of donor tissue, it is contacted with a photosensitizing agent. The term "donor tissue" encompasses any types of transplantable or implantable tissue from a donor other than the recipient that contains APC's. The donor tissue being used in the invention may be any one of a wide variety of tissues such as, for example, soft tissue such as the amniotic membrane of a newborn, bone marrow, hematopoietic precursor cells, collagen, and bone protein to stimulate cartilage growth, organs such as skin, heart, liver, spleen, pancreas, thyroid lobe, lung, kidney, tubular organs (e.g., intestine, blood vessels, or esophagus); and parts of organs such as heart valves and isolated cells or clusters of cells, such as islet cells of the pancreas or liver cells.

Tubular organs can be used to replace damaged portions of esophagus, blood vessels, or bile duct. Skin grafts can be used, not only for bums, but also as a dressing for a damaged intestine or to close certain defects such as a diaphragmatic hernia. In a particularly preferred embodiment, the donor tissue is skin tissue or pancreatic islet cells.

The term "graft" as used herein refers to biological material derived from a donor for transplantation into a recipient. The term "transplant" and variations thereof refers to the insertion of a graft into a recipient, whether the transplantation is syngeneic (where the donor and recipient are genetically identical), allogeneic (where the donor and recipient are of different genetic origins but of the same species), or xenogeneic (where the donor and recipient are from different species). Thus, in a typical scenario, the host is human and the graft is an isograft, derived from a human of the same or different genetic origins. In another scenario, the graft is derived from a species different from that into which it is transplanted, including animals from phylogenically widely separated species, for example, a baboon heart being transplanted into a human host.

The donor tissue can be taken from any source, whether from cadavers or living donors. Examples of suitable donors include live animals such as laboratory animals, for example, dogs, cats, mice, rats, gerbils, guinea pigs, cows, primates, or human beings. Donors are preferably mammalian, including human beings.

Human donors are preferably voluntary, blood-related donors that are normal on physical examination and of the same major ABO blood group, because crossing major blood group barriers can prejudice the survival of an allograft. It is, however, possible to transplant, for example, a kidney of a type O donor into an A, B or AB recipient.

The term "allograft" refers to cells and tissue that originate with or are derived from a donor of the same species as the recipient. Preferably, the donor is of the same species as the recipient.

The term "recipient" as used herein refers to any compatible transplant host. By "compatible" is meant a host that will accept the donated graft. Examples of potentially useful recipients includes animals, preferably mammals such as farm animals, for example, horses, cows or sheep; household pets, for example, dogs or cats; laboratory animals, such as mice, rats, gerbils or guinea pigs; or primates, for example, apes or human beings. Most preferably, the recipient is a human being. If both the donor of the graft and the host are human, they are preferably matched for HLA class II antigens to as to improve histocompatibility.

### Photosensitizing Agents

In general, any one of a great variety of compounds that are useful in classical photodynamic therapy are suitable for use in the present invention. As is well known to those of ordinary skill in this art, a major class of known photosensitizing agents are porphyrin-related compounds. As described in some detail above, these drugs include hematoporphyrin derivatives; the high molecular weight fraction of hematoporphyrin derivative, marketed as Photofrin n photosensitizing composition and the active components thereof, various synthetic derivatives of porphyrins, such as the monohydrobenzoporphyrins, also referred to as benzoporphyrin-derivatives or BPD's; green porphyrins; and various other polycyclic compounds believed to generate singlet oxygen when irradiated, thus causing tissue destruction. Methods for the preparation of suitable photosensitizing compounds are fully disclosed in the patents described above, and in the publications cited therein. Preferably, the photosensitizing agent is BPD.

In principle, the critical feature of any photosensitizing agent is its propensity, when exposed with light of a wavelength capable of being absorbed by the photosensitizer, to exhibit a cytotoxic effect on cells in which it is localized. While it is believed that, in many instances, the cytotoxic effect is a result of the formation of singlet oxygen upon exposure, the exact mode of operation is not critical to the present invention.

As discussed at some length in the aforementioned Dougherty *et al*. patents, a number of additional specific properties are typically associated with effective photosensitizing agents. Among the properties of photosensitizers in general that are of particular significance in the practice of the present invention are a relative absence of toxicity to cells in the absence of the photochemical effect and a ready clearance from tissues in the absence of a target-specific interaction between particular cells and the photosensitizer.

The photosensitizing agents of the present invention preferably have an absorption spectrum which is within the range of wavelengths between 350 nm and 1200 nm, which absorption spectrum may be tailored to the desired penetration in a manner known *per se*, preferably between about 400 and 900 and, most preferably between 600 and 800.

The photosensitizing agents of the invention are dosed in a fashion consistent with good medical practice, taking into account the nature of transplantation and the disorder to be treated, the species of the donor, the medical condition of the individual recipient, the presence of any other drug in the donor tissue or in the recipient's body, and other factors known to practitioners. A therapeutically effective amount of photosensitizer used to contact the graft is an amount that is effective to reduce the immunogenicity of the graft so that it will be compatible with the recipient and not be rejected. The generally effective amount for this purpose is in the range of from 0.1 to 2.0µg/mL and , preferably from about 0.25 to about 1.0 µg/mL.

The photosensitizing agent may be combined with one or more immunosuppressive agents to enhance the immunosuppressant effect on the graft. The effective amount of such other agents depends on the amount of the photosensitizing agent present in the formulation, the type of transplant, the cause of the transplant, the site of delivery, the method of administration, the scheduling of administration, other factors discussed above, and other factors known to practitioners.

Typically, the photosensitizing agent is formulated by mixing it, at ambient temperatures, appropriate pH's, and the desired degree of purity, with one or more physiologically acceptable carriers, i.e., carriers that are non-toxic to recipients at the dosages and concentrations employed. The pH of the formulation depends mainly on the particular use, and concentration of photosensitizer, but preferably ranges anywhere from about 3 to about 8.

Preferably, the photosensitizer is maintained at neutral pH (e.g., about 6.5 to about 7.5) to prevent its adhering to the contains in which it is placed, as occurs at pH values approaching physiological levels, and to ensure activation of the photosensitizer. Thus, formulation in an electrolyte solution containing a balanced salt buffer at pH 6.5, but containing no fetal bovine serum ("FBS"), is a suitable embodiment. The reason the FBS is omitted is because it contains antigenic components which could exacerbate the allograft reaction. If the photosensitizing agent adheres to the containers in which the grafts are being treated, an appropriate non-antigenic ingredient, such as human serum albumin, is optionally added in an amount that does not interfere with the photosensitizing agent from perfusing or adhering to the graft being treated.

If the photosensitizing agent formulation is to be applied topically, for example, if it is to be painted onto a skin graft prior to transplantation, it is preferable to use a viscous solution such as a gel, rather than a non-viscous solution. The gel may be accomplished, for example, by mixing the solution of the photosensitizing agent with a gelling agent, such as a polysaccharide, preferably a water-soluble polysaccharide, such, e.g., hyaluronic acid, starches, and cellulose derivatives, e.g., methylcellulose, hydroxyethyl cellulose, and carboxymethyl cellulose. When a polysaccharide is present in a gel formulation, the amount usually present is in the range of about 1-90% by weight of the gel, more preferably about 1-20%. Examples of other suitable polysaccharides for this purpose, and a determination of the solubility of the polysaccharides, are found in EP 267,015 published 11 May 1988, the disclosure of which is incorporated herein by reference.

If the graft to be treated is to be stored for any period of time, the photosensitizing agent is preferably formulated in or added to a perfluorochemical emulsion (acting as a blood substitute) to enable high concentrations of oxygen to reach the graft. Such emulsions comprise a perfluorochemical such as perfluorodecalin and/or perfluorotripropylamine emulsified with a surfactant in water. The perfluorochemical is chosen to be the least toxic to the recipient.

Examples of suitable surfactants include the poloxamer surfactants, which represent a series of molecules that are block copolymers of ethylene oxide and propylene oxide, either alone or taken in admixture with a phospholipid such as egg lecithin. Another example of an emulsion commercially available from Green Cross is Fluosol-DA 20%, which contains perfluorodecalin and perfluorotripropylamine emulsified with the poloxamer surfactant, Pluronic F-68. The perfluorochemical emulsions and their effects in mammals are described more fully in Bollands et al., J. Pharm. Pharmacol., 39:1021-1024 (1987).

The photosensitizer formulation for use in therapeutic administration is preferably sterile. Sterility is readily accomplished by sterile filtration through (0.2 micron) membranes. Once formulated and sterilized, the photosensitizer may not be stable to oxidative denaturation. However, lyophilized formulations for reconstitution, for example, containing BPD, are suitable for storage.

### Targeting Systems

The use of these photosensitizing agents in destroying the ability of donor tissue to initiate a graft-versus-host reaction is enhanced by conjugation of the photosensitizer to a target-specific agent. In particular, the photosensitizing material may be conjugated to (1) a moiety that specifically targets the antigen-presenting cells (APC's) in the donor tissue directly; (2) a moiety that specifically targets an intermediary material, which labels the APC's for targeting by the conjugate; or (3) T-cells to graft vs. host situation. In either case, once the donor tissue has been modified by interaction with the conjugate, it is exposed in a manner known *per se* so as to effect a substantial depletion of the pool of APC's in the donor tissue.

This invention provides specific, photosensitizer-containing conjugates useful to target APC's in allograft donor tissue, as well as a method to destroy APC's in donor tissue by photodynamic therapy ("PDT") generally. One formulation useful in this process consists essentially of a photosensitizing agent and a system for linking a "homing agent" with the photosensitizer. Another formulation comprises the combination of an APC-targeting system and a photosensitizing agent conjugated with a homing agent for the APC-targeting system. With either formulation, the ultimate objective of delivering the photosensitizing drug to the APC's is identical.

The targeted APC's can be accessed by a variety of different types of target-specific agents, including moieties immunospecific for the MHC glycoprotein products and lymphokine factors for which these cells bear receptors. Typically, for reaction with the MHC glycoproteins, antibodies raised against these glycoproteins, either polygonal or monoclonal, may be used.

Polyclonal antisera are prepared in conventional ways, for example by injecting a suitable mammal with antigen to which antibody is desired, assaying the antibody level in serum against the antigen, and preparing anti-sera when the titers are high. Monoclonal antibody preparations may also be prepared conventionally, such as by the method of Koehler and Milstein, Nature, Vol 256, pp. 495-497 (1975), "Continuous Cultures of Fused Cells Secreting Antibody of Predefined Specificity" and *European Journal of Immunol*., Vol 6, pp. 511-519 (1976) "Derivation of Specific Antibody-Producing Tissue Culture and Tumor Lines by Cell Fusion", using, e.g., peripheral blood lymphocytes or spleen cells from immunized animals and immortalizing these cells either by viral infection, by fusion with myelomas, or by other conventional procedures, and screening for the production of the desired antibodies by isolated colonies.

In addition to antibodies, suitable immunoreactive fragments may also be employed, such as the Fab, Fab', or F(ab)2 fragments. Many antibodies suitable for use in forming the targeting mechanism are already available in the art. For example, the use of immunologically reactive fragments as substitutes for whole antibodies is described by E.L. Morgan et al., *Scand*. *J*. *Immunol*., Vol 10, pp. 395-402 (1979), "Comparison of the Binding of Radiolabelled Human IgG and Fc Fragments to Murine Spleen Cells" and Hans P. Kocher et al., *The Journal of Immunol*., Vol 122, No. 4, pp. 1190-1195 (1979), "Tryptic Degradation of the CH1 and VL *Regions of OgD* and IgE1".

In addition to immunoreactivity, targeting can be effected by using receptor ligands that target receptors at the APC cell surface, for example, on the basis of complementarity of contours or charge patterns between the receptor and ligand. As used herein, the term "receptor ligand" refers to any substance, natural or synthetic, that binds specifically to an APC cell surface receptor. These receptor ligands include lymphokine factors, for example, IL2.

In accordance with a particular embodiment of the present invention, the photosensitizing agent is conjugated with a target-specific agent for an intermediary which, in turn, is specific for the APC. For example, in the case of rat cells presenting Ia, mouse anti-rat Ia antibody could be used as an intermediary. In this case, a conjugate of photosensitizing agent coupled with anti-mouse antibody would target cells labeled with the murine antibody in precisely the same manner as a conjugate comprising anti-rat Ia antibody would do directly.

### Conjugation Methods

The targeting system can be conjugated directly to the photosensitizing drug using conventional methods and linker technology, as are generally known in the art and described by way of example in the aforementioned Levy *et al*. patents. For proteins such as Ig and other polypeptides, a direct covalent bond may be effected between the photosensitizing agent and the target-specific component using, e.g., a dehydrating agent such as a carbodiimide. Active moieties of the conjugate may, of course, also be joined through the use of linker compounds that are bifunctional and capable of covalently binding with each of the two active components.

Any effective technique known in the art to be suitable for joining two chemical moieties falls within the scope of the invention. The linker moiety is to be broadly understood as being a covalent bond or any linker moiety available in the art or derivable therefrom using standard techniques.

Alternatively, the targeting can be mediated by additional specific agents. For example, as illustrated below, a secondary antibody directed to the APC-specific antibody may be linked directly to a photosensitizer, and the MHC-glycoprotein targeting agent may be used as a bridge between the immunoconjugate and the targeted cell.

### Treatment Protocol

Elimination or functional attenuation of APC's, or modulation of other skin cells such as keratinocytes, in accordance with the present invention is effected in a relatively straightforward manner by contacting donor tissue directly with the photosensitizing agent, which may be in conjugate form, under conditions that enable the formation of a strong association between the photosensitizing agent (or the target-specific component of a photosensitizer-containing conjugate) and the target APC's, while minimizing the concentration of the photosensitizer in donor tissue.

The contact suitably involves applying the composition to one or more surfaces of the graft, or incubating or perfusing an organ graft, with the photosensitizer formulation of the invention. The treatment generally takes place for at least one minute, preferably from about 1 minute to about 72 hours, and even more preferably from about 2 minutes to about 24 hours. The time of contact depends on such factors as the concentration of the photosensitizing agent in the formulation, the graft to be treated, and the particular type of formulation. Perfusion is accomplished by any suitable procedure. For example, an organ can be perfused via a device that provides a constant pressure or perfusion having a pressure regulator and overflow situation between a pump and the organ. Alternatively, the organ may be placed in a hyperbaric chamber via a sealing door, and perfusate delivered to the chamber by a pump that draws the fluid from a reservoir, optionally while spent perfusate is return to the reservoir by a valve.

For skin grafts, the formulation can be painted or sprayed onto the lower surface of the skin to be grafted, so that there is a layer of the photosensitizer between the lower surface of the donor and the tissue of the recipient. Preferably, however, the whole skin graft is submerged in the photosensitizer composition.

The contacting step can take place over a wide variety of temperatures, avoiding only those temperatures great enough to denature or otherwise deleteriously affect the graft and those temperatures low enough to minimize cellular uptake of the photosensitizer. Preferably, the contacting step takes place at a temperature in the range from about 50C to about 400C, preferably, from about 150C to about 370C and, most preferably, at ambient temperature.

Following an appropriate distribution of the photosensitizer to ensure that it is properly associated with the target APC's, the thus-treated donor tissue is subjected to exposure with light having a wavelength that is absorbed by the photosensitizing agent and leads to activation of the photosensitizer's cytotoxic properties. Such exposure is, of course, entirely conventional in the art of photodynamic therapy. Exemplary methods and apparatus for this purpose are described, for example, in the aforementioned Dougherty *et al*. patents.

After the graft has been contacted with photosensitizer and exposed to light, it can be stored for as long as about 24-48 hours. Preferably, however, it is used immediately in a transplant procedure. Storage life can be enhanced as described above by using a blood substitute in the formulation (e.g., a perfluorochemical emulsion), or by perfusing the graft with a formulation of the photosensitizing agent containing chilled isotonic agent and anticoagulant, followed by glycerol, to allow for the freezing of grafts with little destruction of the cells, as described in JP 60061501 published 9 April 1985. In addition, the graft can be preserved with different liquids that include the formulation while the organs are being cooled to freezing temperatures, to preserve the organ semi-permanently without cell necrocytosis.

Before transplantation, the graft is preferably washed free of the photosensitizing agent composition, as by soaking it in a physiological saline solution or by other means appropriate for this purpose. Also, prior to transplantation, the recipient may be given one or more donor-specific blood transfusions with PDT-treated peripheral blood monouclear cells to aid in graft survival. An alternative procedure is to subject the recipient to total lymphoid irradiation prior to the transplantation operation. Any other pre-transplant procedures that would be beneficial to the particular transplant recipient can be performed as part of the method of this invention.

In some instances, it is desirable to modify the surface of the graft so as to provide positively or negatively charged groups, as by using a suitable amino acid or polymer or by attaching a physiologically acceptable source of charged functional groups. For example, a negatively charged surface is appropriate for blood vessels to diminish blood clotting. It also is desirable in certain circumstances to render the surface hydrophobic or hydrophilic by coupling, e.g., phenylalanine, serine, or lysine, to the surface. An immunosuppressive agent particularly effective for these surface modifications is glutaraldehyde.

The transplantation procedure itself will depend on the particular disorder being treated, the condition of the patient, etc. The medical practitioner will recognize the appropriate procedure to use in any given case. The transplants are optionally monitored systematically during the critical post-operative period (the first three months) using any suitable procedure, such as radionuclide intravenous angiography. After the transplantation, immunosuppression therapy, using an appropriate immunosuppressant, is often used as important in ensuring graft survival.

The method of the invention can be supplemented by or used in combination with the same or reduced dosages of immunosuppressive agent simultaneously administered to the donor systemically, the donor tissue in vitro, or the recipient, either locally or systemically. The term "immunosuppressive agent" as used herein refers to substances that act to suppress or mask the immune system of the host into which the graft is being transplanted. This would include substances that suppress cytokine production, down-regulate or suppress self-antigen expression, or mask the MHC antigens.

Examples of such agents include 2-amino-6-aryl-5-substituted pyrimidines, azathioprine or cyclophosphamide, bromocryptine, glutaraldehyde, antiidiotypic antibodies for MHC antigens, cyclosporin A, one or more steroids, preferably corticosteroids and glucocorticosteroids such as prednisone, methyl prednisolone, and dexamethasone; anti-interferon-gamma antibodies; anti-tumor necrosis factor-alpha antibodies; anti-tumor necrosis factor-beta antibodies; anti-interleukin-2 antibodies; anticytokine receptor antibodies such as anti-IL-2 receptor antibodies; heterologous anti-lymphocyte globulin; pan-T antibodies, preferably OKT-3 monoclonal antibodies; antibodies to CD4; streptokinase, streptodornase; or RNA or DNA from the host.

An effective amount, which is determined by these considerations, is the minimum amount necessary to prevent an immune response that would result in rejection of the graft by the recipient, but as much as necessary to achieve a longer graft survival time. Such amount is preferably below the amount that is toxic to the recipient or renders the recipient significantly more susceptible to infections. The amount of immunosuppressive agent required for the invention is typically lower than that normally required for transplanted grafts that have not been pre-treated, and depends on the individual circumstances surrounding the transplant and the type of immunosuppressive agent being used.

As a specific example, the total pharmaceutically effective amount of the immunosuppressive agent, cyclosporin A, administered parenterally per dose will be in the range of about 0.1 to 20 mg/kg of patient body weight per day, as compared with the typical range of about 5 to about 15 mg/kg/day cyclosporin A currently used in conventional immunosuppressive therapy. For renal transplants, the usual practice is to administer massive doses of glucocorticosteroid at short periods, e.g., methylprednisolone in several-gram doses per day given for 3 to 5 days, followed by 20 to 100 mg prednisone, without photodynamic pre-treatment of the graft tissue. With the pre-treatment of the invention, significantly lower doses would be useful.

As noted above, these suggested amounts of immunosuppressant are subject to a great deal of therapeutic discretion. The key factor in selecting an appropriate dose and scheduling is the result obtained, i.e., graft survival long-term. For example, relatively high does may be needed either initially for the treatment of hyperacute graft rejection, which can be attributed to antibody-mediated graft destruction, or at a later stage characterized by a sudden decline in graft function.

When an immunosuppressive agent is used, it may be administered by any suitable means, including parenteral, and, if desired for local immunosuppressive treatment, intralesional administration. Parenteral infusions include intramuscular, intravenous, intraarterial, intraperitoneal, or subcutaneous administration. In addition, when an immunosuppressive agent is used, it is suitably administered by pulse infusion, particularly with declining doses, or by continuous infusion.

The following examples are meant to illustrate, but not to limit the invention:

### Example 1

### Preparation of a BPD-Ra-MIq Conjugate

The photosensitizing agent BPD-MA shown in Figure 1 is diluted, in the dark, from a concentration of 1 mg/ml to 200 pg/ml in phosphate-buffered saline and mixed with a known quantity of rat anti-mouse Ig (RaMIg), which is obtained from Cedar Lane Laboratories or prepared by immunizing rabbits with mouse immunoglobulin and purfying the antibodies over immunoabsorbent columns. The mixture is incubated at room temperature for one hour in the dark, and the resulting conjugate is dialyzed overnight through a membrane permeable to molecules having a molecular weight of less than 12-14 kd against three liters of PBS at 4/C. Model studies with labeled BPD-MA show that the retained conjugate has a BPD:Ab ratio of 10-20. The retentate from the dialysis is then frozen and lyophilized and stored in the dark.

### Example 2

### Treatment of Allograft Tissue with the Conjugate

Donor pancreatic islet tissue was isolated from rats as follows:
Male SD rats (200-250 g) were anaesthetized with intraperitoneal urethane (100 mg/kg) and, through a midline laparotomy, cardiorespiratory arrest was induced with bilateral pneumothoraces. The proximal common bile duct was cannulated and distally occluded at its point of entry into the duodenum. The pancreas was then distended in a retrograde fashion with cold (4/C) collagenase solution (Type XI, Sigma Chemicals) at a concentration of 0.42 mg (650 U.) per ml. After in situ collagenase distension, a total pancreatectomy was performed.
The glands were digested for 22 minutes in a 37/C water bath. The digested glands were dispersed by trituration through a sterile, siliconized pipette. The crude tissue slurry was passed through a 200 micron screen filter to remove undigested ducts, blood vessels and lymph nodes, and was then centrifuged through a discontinuous dextran gradient consisting of two monolayers of specific gravity 1.065 and 1.031, respectively. The less dense islet tissue was aspirated from the monolayer interface, washed, and further purified by hand picking under a dissecting microscope. Using this technique, 300-400 functionally and morphologically intact islets were harvested per pancreas.
The islets were cultured in vitro for one day in Ham's F-12 medium supplemented with 25% calf serum, 15 mM HEPES buffer and 1% strepfungizone. The cultured islets were initially incubated with a commercially available mouse anti-rat Ia monoclonal antibody (designated OX-6), obtainable from SeraLab at 0.2 mg/ml for hours at /C. OX-6 is immunospecific against class II MHC product.
Portions of the OX-6-treated islets were incubated with, respectively, (1) Ra-MIgG-BPD conjugate having a ratio of 6.5 BPD:1Ab; (2) the same conjugate at a ratio of 20 BPD:1Ab; (3) a conjugate of BPD with the irrelevant antibody GA-7sIgG at 6.5 BPD:1Ab; (4) BPD alone; and (5) medium alone at 20/C for two hours in the dark. The incubation mixtures were then exposed to 10 Joules/cm² of light energy at a wavelength of 400-800 nm. The irradiated cultures were then tested histologically and for APC depletion.

### Example 3

### Characterization of Donor Tissue

In the histological studies, about 75-100 islets that had been treated with the conjugate of 6.5 BPD:1Ab were transplanted under the kidney capsule of recipient syngeneic SD rats and allogeneic WF rats. In both the syngeneic and allogeneic transplants, all recipients gave successful results. In particular, there was observed complete replacement of the graft with lymphocytic infiltrate and no identifiable endocrine tissue in both syngeneic and allogeneic transplants.

In the APC depletion studies, the islets were primarily immunostained with the OX-6 antibody. The thus-treated cells were subjected to a secondary staining with FITC-labeled goat anti-mouse Ig (Jackson Laboratories) and subjected to fluorescent microscopy. In preparations that had been treated with the conjugates, no identifiable MHC class II cells were seen. In the controls (conjugate with irrelevant antibody, BPD alone, and medium), however, the presence of these cells was detected by fluorescence microscopy, since the FITC-labeled secondary antibody labeled the APC and emitted green fluorescence.

### Example 4

### Prevention of Skin Allograft Rejection

To establish a baseline representing minimal rejection, nine syngrafts (donor and recipient being the same animal) were performed on BALB/c mice, in accordance with standard procedures (Billingham et al., "The Technique of Free Skin Grafting in Mammals", J. Exp. Biol., 28:385-99 (1951)), as follows: The truncal skin of the mouse being grafted was shaved and depilated. The mouse was then anaesthetized by intraperitoneal injection of a mixture of 20 Tl ketamine hydrochloride, 10 Tl xylasine, and 70 Tl PBS, following which a full thickness of skin (1 cm x 1 cm) was obtained by careful dissection, leaving a suitable graft bed and taking care to keep the panniculus carnosus intact.

The autologous skin grafts were then re-applied to the grafting site and held with in place by applying a few about four drops of Vetbond tissue adhesive to the interface between the graft and graft bed. The graft was pressed down with petroleum jelly-coated gauze sponges, and the graft and sponges were held in place with Vetrap bandaging tape, which was wrapped around the body to form a "body cast."

The success rate for long-term syngrafts, i.e., over 120 days, was greater than 90%. Graft rejection was considered complete when there was at least 80% necrosis of the graft. Graft survival was expressed as a mean in terms of survival time in days ± the standard deviation.

Allogeneic skin transplants between C57BL/6 (donors, H-2^{b}) and BALB/c (recipients, H-2^{d}) mice were also performed as controls, using the same procedures as described above except that each skin graft was taken from a donor mouse and applied to the graft bed of a different recipient mouse. Briefly, the truncal skin of donor mice was shaved and depilated, following which full skin grafts (1 cm x 1 cm) were obtained. The recipient mice were shaved and then anaesthetized by intraperitoneal injection of a mixture of 20 Tl ketamine hydrochloride, 10 Tl xylasine, and 70 Tl PBS. The graft bed of each recipient was prepared by careful dissection of the truncal skin (1 cm x 1 cm), taking care to keep the panniculus carnosus intact. The grafts were applied to the allogeneic grafting site, and held with in place by applying Vetbond tissue adhesive, petroleum jelly-coated gauze sponges, and Vetrap bandaging tape, to form a "body cast." The mean survival time was 11.1 days (1.9 standard deviation).

In accordance with the method of the invention, the skin sample to be grafted on a recipient was first contacted in vitro with a 1.0 Tg/mL solution of the photosensitizing agent BPD for one hour. The skin was then suspended in an electrolyte solution containing no fetal bovine serum ("FBS") for 30 minutes and exposed to the red light of light-emitting diodes ("LEDs") (10 J/cm² at 690 nm ± 10 nm). Following this light treatment, the exposed skin was transplanted to a recipient mouse as described above. The animal was monitored for rejection from day 8 following transplantation. The mean survival time for the allografts increased to 18.5 days (2.1 standard deviation). These results are tabulated below in Table I for ease of comparison:

**TABLE 1**

| Type of Graft | Number of Test Animals | Mean Survival Time (Standard Deviation) |
|---|---|---|
| Syngraft | 9 | Indefinite |
| Allografts | 16 | 11.1 days (1.9) |
| Allograft with pre-treatment of donor skin | 6 | 18.5 days (2.1) |

The results suggested that the immunomodulatory effect of photodynamic treatment on tissue to be grafted could result in a significantly extended engraftment survival time.

The experiment was repeated to again compare allografts performed according to the invention, varying the concentration (from 0.25 to 1.0Tg/mL) of the photosensitizing agent BPD, with standard, control allografts. The results of these tests are summarized below in Table 2:

**TABLE 2**

| Type of Graft (No. of Test Animals) | Photosensitizer and Light Dosage | Mean Survival Time (Standard Deviation) |
|---|---|---|
| Control allograft (10) | 0 | 7.5 days (0.84) |
| Allografts with pre-treatment of donor skin (5) | 1.0 Tg/mL BPD, 10 J/cm² LEDs | 11.0 days (0.0) |
| Allografts with pretreatment of donor skin (5) | 0.5 Tg/mL BPD, 10 J/cm² LEDs | 14.3 days (1.5) |

Because it appeared that the two-fold escalation of photosensitizing agent did not itself significantly lengthen the survival time of engraftment, it was postulated that the immunomodulatory effects of photodynamic treatment of tissue to be grafted may depend upon the selective effects on cell populations in the skin and may not necessarily be due to the known cytotoxic effect of photodynamic therapy.

### Example 5

### Histological Examination of Skin to be Grafted

To examine the effects of treating skin grafts with photodynamic therapy under conditions that result in prolonged survival times, skin samples were obtained and treated as described above except with a different range of photosensitizing agent concentration, i.e., 0.25 or 0.50 Tg/mL of BPD. Some tissues were incubated in electrolyte solution alone, without any photosensitizer being present, as control samples. All tissue samples were incubated over a 24-hour period, after which a representative number was exposed to light. When given, light exposure was with red light at an energy level of 10 J/cm².

All samples were then placed in formalin and subjected to histological examination. Tissues incubated in electrolyte solution alone (control samples) or in a solution of 0.50 Tg/mL of BPD, without being light exposed, appeared to be normal. However, samples treated with either 0.25 or 0.50 Tg/mL of BPD, followed by treatment with red light, exhibited the following changes: nuclear enlargement, perinuclear vacuolation, decrease in eosinophilia of epithelial cells, increase in cytoplasmic volume, and increased intercellular spaces between keratinocytes on the epithelial surface. It was postulated that, based on these histological findings, the photodynamic treatment of the invention resulted in a degree of cell damage, rather than cell death. The mechanism for this unexpected, noncytotoxic effect was not known.

It will be clear to those skilled in the art that modifications and/or variations of the disclosed subject matter can be made without departing from the scope of the invention claimed below.

## Claims

1. Use of a photosensitizing agent to prepare a medicament for diminishing the immunogenicity of donor tissue containing antigen presenting cells (APC's) during photodynamic therapy, under conditions which are not cytotoxic to said APC's, using an amount of photosensitizing agent of 0.1 to 2µg/ml.

2. Use as claimed in claim 1, wherein said medicament is for a method which comprises:
a. contacting the donor tissue with a photosensitizing agent to obtain a modified donor tissue;
b. exposing said modified donor tissue to light having a wavelength absorbed by said photosensitizing agent for a time sufficient to functionally attenuate the APC's in the donor tissue; and
c. transplanting donor tissue containing modulated APC's into the tissue of a recipient.

3. A method of treating a donor tissue containing antigen presenting cells (APC's), which method comprises:
(a) contacting the donor tissue with a photosensitizing agent to obtain a modified donor tissue, and
(b) exposing said modified donor tissue to light having a wavelength absorbed by said photosensitizing agent for a time sufficient to functionally attenuate the APC's in the donor tissue, under conditions which are non-cytoxic to said APC's and using an amount of photosensitizer agent of 0.1 to 2µg/ml

4. A method as claimed in claim 3, wherein said donor tissue is human tissue.

5. The use or the method as claimed in any one of claims 2 to 4, wherein at least some of the wavelengths of said light are in the visible portion of the electromagnetic spectrum.

6. The use or the method as claimed in any one of claims 2 to 5, wherein, during said exposing step b, said modified donor tissue is suspended in an electrolyte solution.

7. The use or the method as claimed in any one of claims 2 to 6, wherein the dose of said light during said exposing step b is about 10 J/cm².

8. The use or the method as claimed in any one of the preceding claims, wherein the donor tissue is skin tissue or pancreatic islets.

9. The use or the method as claimed in any one of the preceding claims, wherein said photosensitizing agent is BPD.

10. The use or the method as claimed in any one of the preceding claims, wherein said photosensitizing agent is in the form of a solution ranging in concentration from 0.25 to 1.0 µg/mL.

11. The use or the method as claimed in any one of the preceding claims, wherein said photosensitizing agent is in the form of a conjugate comprising a target-specific component.

12. The use or the method as claimed in claim 11, wherein said target specific agent comprises an agent that binds specifically with the APC's.

13. The use or the method as claimed in claim 12, wherein said target-specific agent is an antibody, a fragment thereof, or a receptor ligand for a receptor on the surface of the APC's.

14. The use or the method as claimed in claim 12, wherein said target-specific agent comprises an agent that binds to a label, which in turn binds specifically with the APC's.

15. A donor tissue having a reduced susceptibility of allograft rejection, prepared by use of a medicament or a method as defined in any one of the foregoing claims.

16. A composition of matter which comprises a donor tissue from a donor, said donor tissue containing antigen presenting cells (APC's) and being in admixture with a medicament comprising a photosensitizing agent at a concentration of 0.1 to 2µg/ml.

## Patentansprüche

1. Verwendung eines Photosensibilisators zur Herstellung eines Arzneimittels zur Verminderung der Immunogenität von Spendergewebe, enthaltend Antigen-präsentierende Zellen (APC) während der photodynamischen Therapie unter Bedingungen, die für die genannten APC unter Verwendung einer Photosensibilisator-Menge von 0,1 bis 2 µg/ml nicht cytotoxisch sind.

2. Verwendung nach Anspruch 1, worin genanntes Arzneimittel für ein Verfahren beabsichtigt ist, das Folgendes umfasst:
a. Kontaktieren des Spendergewebes mit einem Photosensibilisator zum Erhalt eines modifizierten Spendergewebes;
b. Exposition des genannten modifizierten Spendergewebes gegenüber Licht mit einer Wellenlänge, das von genanntem Photosensibilisator für eine Zeit absorbiert wird, die zur funktionellen Attenuierung der APC im Spendergewebe ausreicht; und
c. Transplantation des Spendergewebes enthaltend modulierte APC in das Gewebe eines Empfängers.

3. Verfahren zur Behandlung eines Spendergewebes, enthaltend Antigen-präsentierende Zellen (APC), welches Verfahren Folgendes umfasst:
(a) Kontaktieren des Spendergewebes mit einem Photosensibilisator zum Erhalt eines modifizierten Spendergewebes und
(b) Exposition des genannten modifizierten Spendergewebes gegenüber Licht mit einer Wellenlänge, das von genanntem Photosensibilisator für eine Zeit absorbiert wird, die zur funktionellen Attenuierung der APC im Spendergewebe, unter Bedingungen, die für die genannten APC nicht cytotoxsich sind und unter Verwendung einer Photosensibilisator-Menge von 0,1 bis 2 µg/ml, ausreicht.

4. Verfahren nach Anspruch 3, worin das genannte Spendergewebe humanes Gewebe ist.

5. Verwendung oder Verfahren nach einem der Ansprüche 2 bis 4, worin sich mindestens einige der Wellenlängen von genanntem Licht im sichtbaren Bereich des elektromagnetischen Spektrums befinden.

6. Verwendung oder Verfahren nach einem der Ansprüche 2 bis 5, worin während des genannten Expositionsschrittes b das genannte modifizierte Spendergewebe in einer Elektrolytlösung suspendiert ist.

7. Verwendung oder Verfahren nach einem der Ansprüche 2 bis 6, worin die Dosis von genanntem Licht während des genannten Expositionsschrittes b ca. 10 J/cm² beträgt.

8. Verwendung oder Verfahren nach einem der vorangehenden Ansprüche, worin das Spendergewebe Hautgewebe oder Pankreasinseln darstellt.

9. Verwendung oder Verfahren nach einem der vorangehenden Ansprüche, worin genannter Photosensibilisator BPD darstellt.

10. Verwendung oder Verfahren nach einem der vorangehenden Ansprüche, worin genannter Photosensibilisator in der Form einer Lösung in einem Konzentrationsbereich von 0,25 bis 1,0 µg/ml vorliegt.

11. Verwendung oder Verfahren nach einem der vorangehenden Ansprüche, worin genannter Photosensibilisator in der Form eines Konjugats, umfassend eine targetspezifische Komponente, vorliegt.

12. Verwendung oder Verfahren nach Anspruch 11, worin genanntes targetspezifisches Mittel ein Mittel umfasst, das spezifisch mit den APC bindet.

13. Verwendung oder Verfahren nach Anspruch 12, worin genanntes targetspezifisches Mittel einen Antikörper, ein Fragment davon oder einen Rezeptorliganden für einen Rezeptor auf der Oberfläche der APC darstellt.

14. Verwendung oder Verfahren nach Anspruch 12, worin genanntes targetspezifisches Mittel ein Mittel umfasst, das an eine Markierung bindet, die wiederum spezifisch mit den APC bindet.

15. Spendergewebe mit einer reduzierten Empfindlichkeit gegen eine Allotransplantatabstoßung, das unter Verwendung eines Arzneimittels oder eines Verfahrens, wie nach einem der vorangehenden Ansprüche definiert, vorbereitet wurde.

16. Zusammensetzung eines Materials, das ein Spendergewebe von einem Spender umfasst, wobei genanntes Spendergewebe Antigen-präsentierende Zellen (APC) enthält und eine Beimischung mit einem Arzneimittel, umfassend einen Photosensibilisator in einer Konzentration von 0,1 bis 2 µg/ml, darstellt.

## Revendications

1. Utilisation d'un agent photosensibilisant pour préparer un médicament destiné à diminuer l'immunogénicité de tissu de donneur contenant des cellules présentant un antigène (CPA) pendant une thérapie photodynamique, dans des conditions qui ne sont pas cytotoxiques auxdites CPA, en utilisant une quantité d'agent photosensibilisant de 0,1 à 2 µg / ml.

2. Utilisation selon la revendication 1, dans laquelle ledit médicament est destiné à un procédé comprenant :
a. la mise en contact du tissu de donneur avec un agent photosensibilisant pour obtenir un tissu de donneur modifié ;
b. l'exposition dudit tissu de donneur modifié à une lumière ayant une longueur d'onde absorbée par ledit agent photosensibilisant pendant une durée suffisante pour atténuer fonctionnellement les CPA dans le tissu du donneur ; et
c. la transplantation du tissu du donneur contenant les CPA modulées dans le tissu d'un receveur.

3. Procédé de traitement d'un tissu de donneur contenant des cellules présentant un antigène (CPA), lequel procédé comprend :
(a) la mise en contact du tissu de donneur avec un agent photosensibilisant pour obtenir un tissu de donneur modifié ; et
(b) l'exposition dudit tissu de donneur modifié à une lumière ayant une longueur d'onde absorbée par ledit agent photosensibilisant pendant une durée suffisante pour atténuer fonctionnellement les CPA dans le tissu du donneur, dans des conditions qui ne sont pas cytotoxiques auxdites CPA et utilisant une quantité d'agent photosensibilisant de 0,1 à 2 µg / ml.

4. Procédé selon la revendication 3, dans lequel ledit tissu de donneur est un tissu humain.

5. Utilisation ou procédé selon l'une quelconque des revendications 2 à 4, dans lesquels au moins une partie des longueurs d'ondes de ladite lumière est dans la partie visible du spectre électromagnétique.

6. Utilisation ou procédé selon l'une quelconque des revendications 2 à 5, dans lesquels, pendant l'étape d'exposition b, ledit tissu de donneur modifié est suspendu dans une solution électrolyte.

7. Utilisation ou procédé selon l'une quelconque des revendications 2 à 6, dans lesquels la dose de ladite lumière pendant ladite étape d'exposition b est environ 10 J / cm².

8. Utilisation ou procédé selon l'une quelconque des revendications précédentes, dans lesquels le tissu de donneur est du tissu de peau ou des îlets pancréatiques.

9. Utilisation ou procédé selon l'une quelconque des revendications précédentes, dans lesquels ledit agent photosensibilisant est du BPD.

10. Utilisation ou procédé selon l'une quelconque des revendications précédentes, dans lesquels ledit agent photosensibilisant revêt la forme d'une solution dont la concentration est comprise dans la plage allant de 0,25 à 1,0 µg / mL.

11. Utilisation ou procédé selon l'une quelconque des revendications précédentes, dans lesquels ledit agent photosensibilisant revêt la forme d'un conjugué comprenant un composant spécifique à une cible.

12. Utilisation ou procédé selon la revendication 11, dans lesquels ledit agent spécifique à une cible comprend un agent qui se lie spécifiquement aux CPA.

13. Utilisation ou procédé selon la revendication 12, dans lesquels ledit agent spécifique à une cible est un anticorps, un fragment de celui-ci ou un ligand de récepteur pour un récepteur sur la surface des CPA.

14. Utilisation ou procédé selon la revendication 12, dans lesquels ledit agent spécifique à une cible comprend un agent qui se lie à une étiquette, qui à son tour se lie spécifiquement aux CPA.

15. Tissu donneur ayant une susceptibilité réduite au rejet d'allogreffe, préparé en utilisant un médicament ou un procédé défini dans l'une quelconque des revendications précédentes.

16. Composition de matière comprenant un tissu donneur provenant d'un donneur, ledit tissu donneur contenant des cellules présentant un antigène (CPA) et étant mélangé avec un médicament comprenant un agent photosensibilisant en une concentration de 0,1 à 2 µg / ml.
